# EUROPÄISCHE PATENTSCHRIFT

(11) **EP 1 335 714 B1**
(45) Veröffentlichungstag und Bekanntmachung des Hinweises auf die Patenterteilung: **26.01.2005**
(21) Anmeldenummer: 01993463.7
(22) Anmeldetag: 31.10.2001
(51) Int. Cl.: A61K 9/70

(54) **HAUTFREUNDLICHES WIRKSTOFFPFLASTER ZUR TRANSDERMALEN VERABREICHUNG ÄTHERISCHER ÖLE**
SKIN FRIENDLY ACTIVE INGREDIENT PLASTER FOR TRANSDERMAL ADMINISTRATION OF ETHERIC OILS
TIMBRE RENFERMANT UNE SUBSTANCE ACTIVE, DOUX POUR LA PEAU, PERMETTANT L'ADMINISTRATION TRANSDERMIQUE D'HUILES ESSENTIELLES

(30) Priorität: 11.11.2000 DE 10056011
(43) Veröffentlichungstag der Anmeldung: 20.08.2003
(73) Patentinhaber: Beiersdorf AG, 20253 Hamburg (DE)
(72) Erfinder: WÖLLER, Karl-Heinz, 20257 Hamburg (DE)
(86) Internationale Anmeldenummer: PCT/EP2001/012604
(87) Internationale Veröffentlichungsnummer: WO 2002/038136

(56) Entgegenhaltungen:
- EP-A- 0 341 202
- EP-A- 0 788 792
- WO-A-00/45797
- WO-A-02/38196

## Beschreibung

Die Erfindung betrifft Transdermale Therapeutische Systeme, welche mit ätherischen Ölen und deren Bestandteilen (zum Beispiel Eucalyptusöl, Pfefferminzöl, Campher, Menthol) dotiert sind und einen langfristigen, therapeutischen Effekt bei Erkältungskrankheiten, Kopfschmerzen und weiteren Indikationen besitzen.

Ätherische Öle und ihre Bestandteile gehen bereits in einem niedrigen Temperaturbereich vom flüssigen in den gasförmigen Zustand über, so daß die durchschnittliche Körpertemperatur ausreichend ist, um ein schnelles Verdunsten der ätherischen Öle herbeizuführen.
Bei einer cutanen Applikation von ätherischen Ölen durch flüssige oder halbfeste Arzneiformen kann so durch Inhalieren und/oder transdermale Effekte lediglich ein kurzzeitiger therapeutischer Effekt erzielt werden, der eine häufige Wiederholung der Applikation zur Folge hat. Eine durchgehende Behandlung des Patienten über einen längeren Zeitabschnitt muß aus diesem Grund mit einer Arzneiform erfolgen, in welcher die ätherischen Öle in einer retardierten Form vorliegen.

Als Lösung für dieses Problem sind Transdermale Therapeutische Systeme (TTS) zu nennen, welche pflasterartige, arzneistoffdotierte Systeme darstellen. Die zeitabhängige Freisetzung des Arzneistoffs aus dem TTS erfolgt in Abhängigkeit seines Verteilungskoeffizienten TTS/Haut und seiner Diffusion im Bereich des TTS und der Haut.
Beide Faktoren können durch die Zusammensetzung der Matrix, in die der Arzneistoff eingearbeitet ist, beeinflußt werden, wodurch die pro Zeiteinheit freigesetzte Arzneistoffmenge und die Dauer der Wirksamkeit direkt beeinflußt werden kann. Im Idealfall wird eine Freisetzungskinetik erster Ordnung erreicht, was eine Freisetzung gleicher Wirkstoffmengen pro Zeiteinheit ermöglicht. Diese Eigenschaften des TTS verhindern eine wiederholte Applikation und Belastung der Haut mit hohen Konzentrationen ätherischer Öle und damit eine Reizung der Haut, die bei wiederholter Applikation von flüssigen und halbfesten Arzneiformen unumgänglich ist. Sollten dennoch Nebenwirkungen im Verlauf einer TTS Applikation auftreten, kann durch das Entfernen des TTS die weitere Belastung durch die ätherischen Öle sofort gestoppt werden.

Durch eine Abdeckung der arzneistoffhaltigen Matrix mit einer Folie, die einen Austritt der ätherischen Öle zur körperabgewandten Seite des TTS verhindert, wird der transdermale Effekt zusätzlich verstärkt. Gleichzeitig wird die Freisetzung von Geruchsstoffen, die Bestandteile der ätherischen Öle sind, reduziert und damit eine unauffällige Applikation des TTS ermöglicht. Bei Erkrankungen der Atemwege ist dieser Aufbau des TTS zu bedenken, da der zusätzliche pharmakologische Effekt, der durch das Inhalieren der ätherischen Öle erreicht wird, unterbunden wird. In diesem Fall kann sogar die Überlegung angestellt werden, ob die Abdeckung der Matrix mit der ätherisch Öl undurchlässigen Folie auf der Körperseite erfolgt. Hierdurch wird der therapeutische Effekt durch das Inhalieren der Arzneistoffe in den Vordergrund gesetzt, während die transdermale Diffusion verhindert wird.

Als abschließender Vorteil des TTS ist die deutlich verbesserte Compliance der Patienten zu nennen, die auf die einfache und schnelle Applikation sowie die lange Wirksamkeit des TTS zurückzuführen ist.

Deutliche Nachteile, die bei der Entwicklung der TTS gelöst werden müssen, ist die Freisetzung des Arzneistoffs aus dem System und eine ausreichende Aufnahme des Arzneistoffs in die Haut, um die therapeutisch wirksame Konzentration zu erreichen. Um diese Parameter steuern zu können, werden Hydrokolloide, Lösungsvermittler und Enhancer eingesetzt, welche eine verbesserte Löslichkeit und Diffusion sowie einen schnelleren Übergang des Arzneistoffs von TTS ins Stratum comeum ermöglichen. Da die Bestandteile der ätherischen Öle Eigenschaften besitzen, die eine schnelle Penetration in den Bereich der Haut bewirken, erfolgt die Applikation TTS in der Regel ohne den Einsatz von Enhancern.

Eine in der Fachliteratur gut beschriebene Ausführungsform solcher transdermalen Systeme stellen Matrixsysteme oder monolitische Systeme dar, in denen der Arzneistoff direkt in den druckempfindlichen Haftklebstoff eingearbeitet wird. Eine solche haftklebrige, wirkstoffhaltige Matrix ist im anwendungsfertigen Produkt auf der einen Seite mit einem für den Wirkstoff undurchlässigen Träger ausgestattet, auf der gegenüberliegenden Seite befindet sich eine mit einer Trennschicht ausgestattete Trägerfolie, die vor der Applikation auf die Haut entfernt wird (kleben&dichten, Nr.42, 1998, S. 26 bis 30).

Eine Grundanforderung an ein TTS ist ein sehr gutes Haftvermögen auf Haut, das über den gesamten Zeitraum der beabsichtigten Wirkstoffdosierung aufrechterhalten bleiben muß. Eine häufig beobachtete Nebenwirkung ist jedoch das Auftreten von Hautirritationen, die besonders bei längerer oder wiederholter Applikation eines TTS an einer gleichbleibenden Körperregion auftreten. Sie werden hauptsächlich durch die Inhaltsstoffe der haftklebrigen Matrix verursacht. Auch ein schmerzhaftes Wiederablösen des wirkstoffhaltigen Pflasters nach längerer Tragezeit wird häufig beobachtet.

Wiederholte und lange andauernde Anwendungen von haftklebrigen Systemen an immer gleichen Regionen des menschlichen Körpers sind vor allem im Bereich der Stoma-Versorgung anzutreffen. Hier werden seit langer Zeit und mit großem Erfolg Hydrokolloide als Haftklebstoff eingesetzt. Diese bestehen prinzipiell aus einer hydrophoben haftklebrigen Polymermatrix auf der Basis synthetischer Kautschuke, in der in dieser Matrix unlösliche hydrophile Füllstoffe auf der Basis von zum Beispiel Alginaten, Cellulose oder Pektinen dispers verteilt vorliegen.
Bei der Entwicklung von Hydrokolloiden zur Stoma-Versorgung stehen jedoch die Hafteigenschaften auf feuchter Haut und die Fähigkeit der Flüssigkeitsaufnahme im Vordergrund.

Bereits 1967 wird in US 3,339,546 ein Hydrokolloid auf der Basis von Polyisobutylenen zur Anwendung im Mundraum beschrieben. Ein großer Nachteil der frühen Systeme besteht in der mangelhaften Integrität der Matrices, d.h. in einem Auflösen und Zerfall der haftklebrigen Matrix bei der Aufnahme größerer Mengen an Flüssigkeit.

Spätere Entwicklungen zielen deshalb auf die Lösung dieses Problems ab und mehrere Lösungsansätze werden in der Literatur aufgezeigt.

US 4,393,080 beschreibt zum Beispiel ein Hydrokolloid-System auf der Basis von Elastomeren, wobei hochmolekulare hydrophile Füllstoffe eingesetzt werden, die einen Zusammenhalt auch des gequollenen Systems fördern.
Weitere Dokumente beschreiben Lösungsansätze über die Vernetzung der Elastomermatrix, die entweder physikalisch oder chemisch verlaufen kann.

Eine physikalische Vernetzung kann zum Beispiel durch den Einsatz phasenseparierender Blockpolymere auf der Basis von Poly(styrol-b-isopren-b-styrol) (SIS), Poly(styrol-bisopren-b-styrol) (SBS) oder Poly[styrol-b-(ethylen-stat.-butylen)-b-styrol] (SEBS) erfolgen. Eines der ersten solcher Systeme wird zum Beispiel in der DE 28 22 535 beschrieben.

Eine chemische Vernetzung kann zum Beispiel über Elektronen- oder γ-Bestrahlung der Hydrokolloidmatrix erfolgen. Hierbei wird vorausgesetzt, daß in der haftklebrigen Matrix genügend reaktive Strukturelemente vorhanden sind. Dies kann beispielsweise, wie in US 4,477,325 beschrieben, über die Compoundierung mit einem Ethylen-Vinylacetat Copolymer erfolgen.

Innerhalb der vorgenannten Erfindungen wird zwar das technische Problem der Kohäsivität gequollener Hydrokolloide beschrieben und gelöst, das Problem der Hautreizung durch wiederholte Anwendung wird jedoch nicht adressiert.

Mit möglicherweise auftretenden Hautreizungen beschäftigt sich hingegen WO 98/01167 A1. Hier wird Aloe Vera Extrakt zur Vermeidung entzündlicher Hautveränderungen sowie Infektionen bei der Stoma-Versorgung eingesetzt. Das beschriebene System besteht jedoch lediglich aus einem niedermolekularen Polyisobutylen als Polymergerüst, wodurch das zuvor beschriebene Problem der Kohäsivität der Hydrokolloid-Matrix weiterhin besteht. Zusätzlich werden in der beschriebenen Zusammensetzung Klebharze eingesetzt, deren allergenes Potential bekannt ist.

Informationen bezüglich der Eignung eines solchen Systems zur kontrollierten Abgabe von Arzneistoffen sind weder dieser noch in einer anderen der genannten Schriften enthalten.

Transdermal Therapeutische Systeme werden in der Regel auf gesunder, intakter Haut appliziert. Gerade hier ist es besonders wichtig, die intakte Haut nicht durch ein Arzneimittel zu reizen oder gar zu schädigen. Eine genügende Kohäsivität ist zusätzlich notwendig, um das wirkstoffhaltige Pflaster nach beendeter Tragedauer rückstandsfrei entfernen zu können.

Polyisobutylene werden seit langer Zeit als Gerüstsubstanz bei der Compoundierung von Haftklebstoffen eingesetzt. Gegenüber anderen bekannten Elastomeren bieten synthetische Polymere auf der Basis von Isobutylen eine Reihe von Vorteilen. Sie sind durch ihre synthetische Herstellung frei von unerwünschten Inhaltsstoffen, durch ihre vollständige Sättigung sind sie sehr oxidationsstabil und sie zeichnen sich je nach Molekulargewicht durch eine einstellbare inhärente Klebrigkeit aus.

Vor allem für die Anwendung auf Haut sind sie deshalb anderen Elastomeren vorzuziehen. So ist zum Beispiel das allergene Potential von Naturkautschuk aufgrund seiner natürlichen Verunreinigungen hinlänglich bekannt. Andere synthetische Kautschuke auf der Basis von Styrol und Isopren beziehungsweise Butadien sind sehr oxidationsempfindlich, wodurch eine aufwendige Additivierung notwendig ist. Ihre hydrierten Derivate auf der Basis von Poly[styrol-b-(ethylen-stat-propylen)-b-styrol] (SEPS) oder Poly[styrol-b-(ethylen-stat.-butylen)-b-styrol] (SEBS) sind zwar oxydationsstabiler, ihnen fehlt es jedoch an inhärenter Klebrigkeit. Aus diesem Grund ist für deren Einsatz als Haftklebstoff zusätzlich die Compoundierung mit Klebharzen unabdingbar, wie beispielsweise in EP 0 651 635 B1 beschrieben. Diese sind in der Regel sehr schlecht definierte Stoffgemische und häufig auf der Basis von Kolophonium. Dadurch ist auch hier ein allergenes Potential nicht auszuschließen.

Der Einsatz von Polyisobutylenen für transdermal Therapeutische Systeme wird in DE 33 47 278 A1 und DE 33 47 277 A1 beschrieben. Hier allerdings immer in der Kombination mit entweder olefinischen Dienkautschuken oder Klebharzen, die wieder die oben beschriebenen Nachteile aufweisen. Auch der Einsatz amorpher Poly-α-olefine als Zusatz wird beschrieben, ohne jedoch deren Einfluß auf das Gesamtsystem zu erläutern. Der Einsatz von Füllstoffen wird in dieser Beschreibung nicht erwähnt.

Der Einsatz von PIB für transdermale Systeme ohne Zusatz von Klebharzen wird in der US 4,559,222 beschrieben. Notwendig ist hier jedoch der Einsatz sehr großer Mengen Mineralöl, wobei das Verhältnis von Mineralöl zu PIB erfindungsgemäß mindestens 1 beträgt. Weiterhin ist das System auf Wirkstoffe beschränkt, die in Mineralöl mäßig löslich sind. Hierdurch wird zusätzlich ein weichmachender Effekt auf die Matrix ausgeübt. Als Füllstoffe werden mindesten 6 Gew.-% kolloidales Silica eingesetzt.

WO 96/22083 A1 beschreibt ein System zur transdermalen Verabreichung von Nikotin, das auf der Basis von Polyisobutylen ohne den Zusatz von Mineralöl auskommt. Der notwendige tack der Klebmasse wird hier jedoch über den Einsatz von Klebharzen erreicht. Diese besitzen hinsichtlich Hautverträglichkeit die oben genannten Nachteile. Ein weichmachender Effekt, der das adhäsive Verhalten der Matrix zusätzlich positiv beeinflußt, wird über den in der PIB Matrix löslichen Wirkstoff erreicht. Dieses Prinzip der Compoundierung schränkt jedoch die Auswahl der über diese Matrix verabreichbaren Wirkstoffe sehr ein.

US 5,508,038 adressiert als einzige der genannten Verbindungen das Problem der Hautreizung durch die mögliche Verwendung von Klebharzen.

Der Einsatz amorpher Poly-α-olefine in Haftklebstoffen im allgemeinen ist in der Literatur bekannt. US 4,186,258 ist eines der ersten Dokumente, die den Einsatz dieser Substanzklasse im Bereich der Schmelzhaftklebstoffe benennt. Ein spezielles Einsatzgebiet wird in dieser Schrift nicht genannt.

US 5,262,216 beschreibt den Einsatz dieser Materialien zusammen mit Klebharzen für Schmelzhaftkleber gezielt für den Einsatzbereich an Selbstklebeetiketten. Besonders ausgelobt wird hier die herausragende UV- und Alterungsbeständigkeit dieser Polymerklasse. Der Einsatzbereich am Menschen wird nicht erwähnt.

WO 98/54268 A1 hingegen beschreibt gezielt den Einsatz amorpher Poly-α-olefine für Anwendungen auf menschlicher Haut. Zusätzlich werden hier amorphe Poly-α-olefine in Kombination mit Füllstoffen verwendet. Allerdings wird speziell der Einsatzbereich der Wundabdeckung beschrieben. Amorphe Poly-α-olefine zeichnen sich in diesem Einsatzgebiet durch ihre hervorragende Strahlenresistenz aus, wodurch erfindungsgemäß gut sterilisierbare Produkte zur Wundversorgung hergestellt werden können. Zusätzlich werden hier amorphe Poly-α-olefine in Kombination mit Klebharzen eingesetzt. Der Aspekt der verminderten Hautreizung wird insgesamt nicht erwähnt. Des weiteren enthält WO 98/54268 A1 keine Anhaltspunkte dafür, daß ein solches System zur Abgabe von Arzneistoffen über die menschliche Haut geeignet ist. Das Einsatzgebiet der transdermal therapeutischen Systeme wird in dieser Schrift vollständig ausgeklammert.

Die bisher beschriebenen Systeme zur transdermalen Verabreichung eines Wirkstoffes enthalten keine organischen Füllstoffe. Jedoch sind insbesondere diese Füllstoffe für die Hautfreundlichkeit der zuvor beschriebenen Haftklebstoffe zur Stoma-Versorgung verantwortlich. Durch sie kann während der Tragedauer des Pflasters von der Haut abgegebene Feuchtigkeit sehr gut aufgenommen werden. Das dadurch entstehende Klima unter dem Pflaster führt zur deutlichen Verminderung des Auftretens von Hautmazerationen.

Eine Erfindung eines Wirkstoffpflasters unter Einsatz von in Wasser quellfähigen Füllstoffen beschreibt EP 0 186 019 A1. Hier wird jedoch der positive Einfluß des organischen Füllstoffes auf die Freisetzungsrate des Wirkstoffes beschrieben. Der erfindungsgemäße Füllstoffanteil ist auf 30 Gew.-% begrenzt. Der Aspekt der Verminderung von Hautreizungen wird nicht angesprochen. Zusätzlich werden die beschriebenen Systeme unter Einsatz von Klebharzen realisiert.

WO 00/45797 A offenbart ein Verfahren zur Herstellung einer Matrix zur transdermalen Verabreichung. In einigen Beispielen werden Transdermalsysteme beschrieben, die Polyisobutylene, Polybuten, Silica, Crospovidone und einen Arzneistoff enthalten und frei von Mineralölen und Klebharzen sind.

Ziel der vorliegenden Erfindung ist es, eine Matrix zur kontrollierten Abgabe eines Arzneistoffes aus der Gruppe der ätherischen Öle zu entwickeln, die sowohl eine ausgezeichnete Kohäsivität besitzt als auch aus besonders hautfreundlichen Komponenten aufgebaut ist beziehungsweise unter vollständigem Verzicht hautreizender Komponenten wie zum Beispiel Klebharze realisiert werden kann. Zusätzlich ist ein Herstellprozeß geplant, der unter vollständigem Verzicht von Lösungsmittel auskommt, des weiteren sollen die genannten Nebenwirkungen eines Haftklebstoffes für transdermale Systeme - Hautirritationen und schmerzhaftes Wiederablösen - vermieden werden, was eine deutliche Steigerung des Tragekomforts für den Patienten ergibt.
Es soll ein Matrixsystem auf der Basis von Polyisobutylen zur Verfügung gestellt werden, das ohne herkömmliche Klebharze und Mineralöl in einem lösungsmittelfreien Herstellprozeß realisierbar ist.

Gelöst wird diese Aufgabe durch ein wirkstoffhaltiges Matrixpflaster gemäß dem Hauptanspruch. Gegenstand der Unteransprüche sind vorteilhafte Ausführungsformen des erfindungsgemäßen Pflasters. Des weiteren umfaßt die Erfindung Verfahren zur Herstellung derartiger Pflaster.

Demgemäß betrifft die Erfindung ein wirkstoffhaltiges Matrixpflaster zur kontrollierten Abgabe von ätherischen Ölen an die Haut und/oder die Atemluft, bestehend aus einer flexiblen Deckschicht und einer wirkstoffhaltigen, wasserunlöslichen haftklebrigen Matrix, wobei die haftklebrige Matrix frei von Mineralölen und Klebharzen ist und aufgebaut ist aus
a) synthetischen Gerüstpolymeren auf der Basis von Polyisobutylen zu 25 bis 90 Gew.-%,
b) amorphem Poly-α-olefin zu 5 bis 30 Gew.-%,
c) aus einem unlöslichen, insbesondere hydrophilen Füllstoff zu 0 bis 60 Gew.-% und
d) ätherischen Ölen zu 0,5 bis 25 Gew.-%.

Unter ätherischen Ölen sind aus Pflanzen gewonnene Konzentrate bekannt, die als natürliche Rohstoffe hauptsächlich in der Parfüm- und Lebensmittelindustrie eingesetzt werden und die mehr oder weniger aus flüchtigen Verbindungen bestehen, wie zum Beispiel echte ätherische Öle, Citrusöle, Absolues, Resinoide.
Oft wird der Begriff auch für die noch in den Pflanzen enthaltenen flüchtigen Inhaltsstoffe verwendet. Im eigentlichen Sinn versteht man aber unter ätherischen Ölen Gemische aus flüchtigen Komponenten, die durch Wasserdampfdestillation aus pflanzlichen Rohstoffen hergestellt werden.

Echte ätherische Öle bestehen ausschließlich aus flüchtigen Komponenten, deren Siedepunkt überwiegend zwischen 150 und 300 °C liegen. Anders als zum Beispiel fette Öle hinterlassen sie deshalb beim Auftupfen auf Filterpapier keinen bleibenden durchsichtigen Fettfleck. Ätherische Öle enthalten überwiegend Kohlenwasserstoffe oder monofunktionelle Verbindungen wie Aldehyde, Alkohole, Ester, Ether und Ketone.
Stammverbindungen sind Mono- und Sesquiterpene, Phenylpropan-Derivate und längerkettige aliphatische Verbindungen.

Bei manchen ätherischen Ölen dominiert ein Inhaltsstoff (zum Beispiel Eugenol in Nelkenöl mit mehr als 85%), andere sind wieder äußerst komplex zusammengesetzt. Oft werden die organoleptische Eigenschaften nicht von den Hauptkomponenten, sondern von Neben- oder Spurenbestandteilen geprägt, wie zum Beispiel von den 1,3,5-Undecatrienen und Pyrazinen im Galbanum-Öl. Bei vielen der kommerziell bedeutenden ätherischen Öle geht die Zahl der identifizierten Komponenten in die Hunderte. Sehr viele Inhaltsstoffe sind chiral, wobei sehr oft ein Enantiomer überwiegt oder ausschließlich vorhanden ist, wie zum Beispiel (-)-Menthol im Pfefferminzöl oder (-)-Linalylacetat im Lavendelöl.

In einer vorteilhaften Ausführungsform enthält die Matrix des Matrixpflasters 2 bis 10 Gew.-% ätherische Öle, die insbesondere aus der Gruppe Eucalyptusöl, Pfefferminzöl, Kamillenöl, Campher, Menthol, Citrusöl, Zimtöl, Thymianöl, Lavendelöl, Nelkenöl, Teebaumöl, Cajeputöl, Niaouliöl, Kanukaöl, Manukaöl, Latschenkieferöl gewählt sind.

Citrusöle sind ätherische Öle, die aus den Schalen von Citrusfrüchten (Bergamotte, Grapefruit, Limette, Mandarine, Orange, Zitrone) gewonnen werden, oft auch Agrumenöle genannt.
Citrusöle bestehen zu einem großen Teil aus Monoterpen-Kohlenwasserstoffen, hauptsächlich Limonen (Ausnahme: Bergamottöl, das nur ca. 40% enthält).

Unter Campher versteht man 2-Bornanon, 1,7,7-Trimethylbicyclo[2.2.1]heptan-2-on, siehe untere Abbildung.

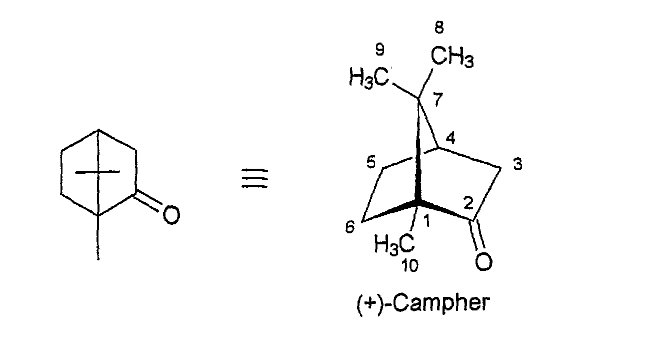

Pfefferminzöle sind durch Wasserdampfdestillation aus Blättern und Blütenständen verschiedener Pfefferminze-Sorten gewonnene ätherische Öle, gelegentlich auch solche aus Mentha arvensis.

Menthol hat drei asymmetrische C-Atome und kommt demzufolge in vier diastereomeren Enantiomerenpaaren vor (vgl. die Formelbilder, die anderen vier Enantiomeren sind die entsprechenden Spiegelbilder).

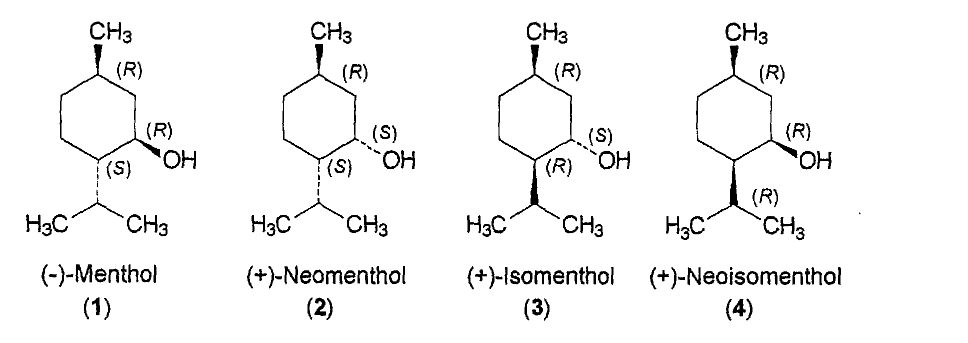

Die Diastereomeren, die destillativ getrennt werden können, werden als Neoisomenthol, Isomenthol, Neomenthol [(+)-Form: Bestandteil des japanischen Pfefferminzöls] und Menthol bezeichnet. Wichtigstes Isomer ist (-)-Menthol (Levamenthol), glänzende, stark pfefferminzartig riechende Prismen.

Menthol erzeugt beim Einreiben auf der Haut (besonders an Stirn und Schläfen) infolge Oberflächenanästhesierung und Reizung der kälteempfindlichen Nerven bei Migräne und dergleichen ein angenehmes Kältegefühl; tatsächlich zeigen die betreffenden Stellen normale oder erhöhte Temperatur. Diese Wirkungen besitzen die anderen Isomeren von Menthol nicht.

In einer weiteren vorteilhaften Ausführungsform des wirkstoffhaltigen Matrixpflasters setzt sich das Polyisobutylen zusammen aus hochmolekularem PIB zu 5 bis 55 Gew.-% und niedermolekularem PIB zu 20 bis 60 Gew.-%.

Eine typischer erfindungsgemäßer Haftklebstoff besteht somit aus folgenden Komponenten:

| | | |
|---|---|---|
| hochmolekulares PIB | 5 - 55 Gew. % | bevorzugt 25 - 45 Gew. % |
| niedermolekulares PIB | 20 - 60 Gew.-% | bevorzugt 30 - 50 Gew. % |
| amorphes Poly-α-olefin | 5 - 30 Gew.-% | bevorzugt 5 -10 Gew. % |
| hydrophiler Füllstoff | 0 - 60 Gew.-% | bevorzugt 0 - 30 Gew. % |
| Ätherisches Öl | 0,05 - 25 Gew.-% | bevorzugt 1,0 -15 Gew.-% |

Optional können noch bis zu 20 Gew.-% eines permeationsfördernden Hilfsstoffes (lipophiler Lösungsvermittler/Enhancer) wie Ölsäuredecylester, Isopropylmyristat und - palmitat (IPM und IPP) zugesetzt werden.

Die genannten Rezepturbestandteile werden dabei wie folgt genauer definiert:

### Hochmolekulares PIB:

Polyisobutylen mit einem gewichtsmittleren Molekulargewicht (M_{w}) von 300.000 bis 1.100.000, bevorzugt zwischen 650.000 und 850.000. Solche Polymere sind kommerziell beispielsweise unter den Handelsnamen Oppanol B100 (BASF) oder Vistanex MM-L80 (Exxon) erhältlich.

### Niedrig molekulares PIB:

Polyisobutylen mit einem gewichtsmittleren Molekulargewicht (M_{w}) von 40.000 bis 300.000, bevorzugt zwischen 60.000 und 100.000. Solche Polymere sind kommerziell beispielsweise unter den Handelsnamen Oppanol B15 (BASF) oder Vistanex LMMH (Exxon) erhältlich.

### Amorphes Poly-α-olefin:

Amorphe Copolymere auf der Basis von Ethylen und Propylen, Butylen oder 1-Hexen. Das bevorzugte gewichtsmittlere Molekulargewicht (M_{w}) liegt bei 5.000 bis 100.000, bevorzugt zwischen 10.000 und 30.000. Solche Polymere sind kommerziell beispielsweise unter den Handelsnamen Eastoflex ® (Eastman) oder Vestoplast ® (Hüls) erhältlich.

### Insbesondere hydrophiler Füllstoff:

In der genannten Polymermatrix unlösliche, hydrophile Partikel auf der Basis von Ceilulose. Bevorzugt ist eine mittlere Partikelgröße von kleiner gleich 100 µm mit einer möglichst gleichförmigen Oberfläche. Solche Materialien sind zum Beispiel unter den Handelsnamen Avicel (FMC) und Elcema (Degussa-Hüls) kommerziell erhältlich.

In einer weiteren vorteilhaften Ausführungsform der Erfindung sind der Matrix hautpflegende, kosmetische Zusatzstoffe zugesetzt, besonders zu 0,2 bis 10 Gew.-%, ganz besonders zu 0,5 bis 5 Gew.-%.

Erfindungsgemäß können die Zusatzstoffe (eine oder mehrere Verbindungen) sehr vorteilhaft gewählt werden aus der Gruppe der lipophilen Zusatzstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Zusatzstoffe aus der Gruppe der rückfettenden Substanzen zu wählen, beispielsweise Purcellinöl, Eucerit® und Neocerit®.

Besonders vorteilhaft werden der oder die Zusatzstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Zusatzstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Zusatzstoff im Sinne der vorliegenden Erfindung.

Vorteilhaft sind femer pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Zusatzstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Zusatzstoffe im Sinne der vorliegenden Erfindung. Sie sind durch folgende Grundstruktur gekennzeichnet (Substitutionspostitionen angegeben):

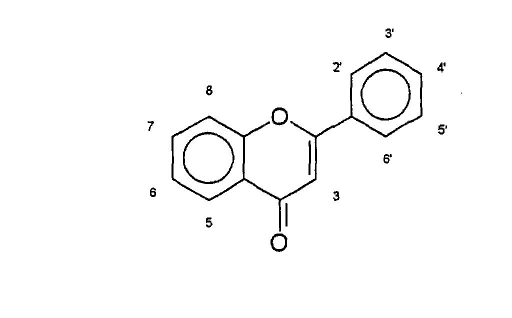

Einige der wichtigeren Flavone, weiche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kampferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel 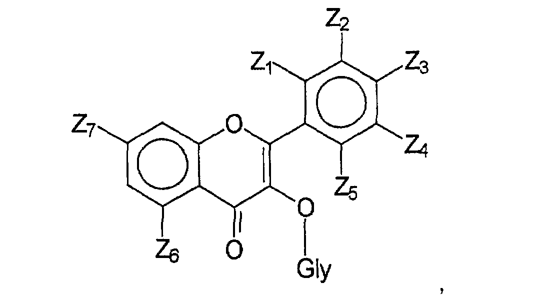 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 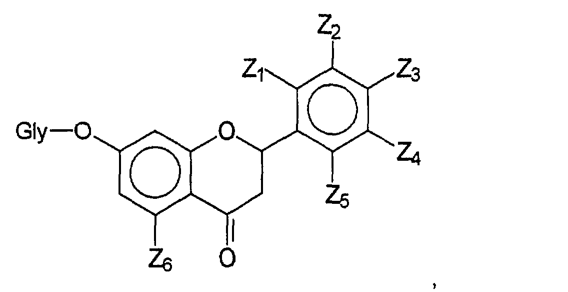 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 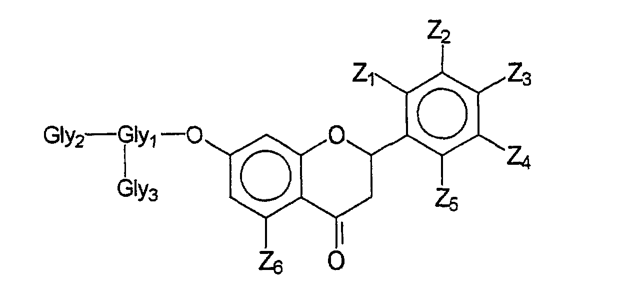 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

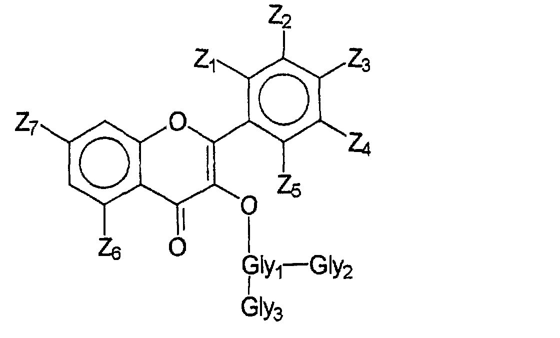

Besonders vorteilhaft werden die erfindungsgemäßen Flavonglycoside aus der Gruppe, welche durch die folgende Struktur wiedergegeben werden: 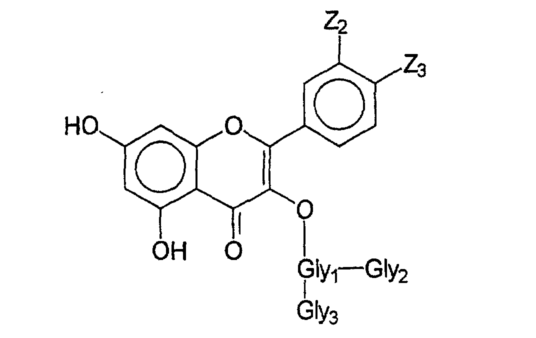 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, den oder die Zusatzstoffe aus der Gruppe der Ubichinone und Plastochinone zu wählen.

Ubichinone zeichnen sich durch die Strukturformel 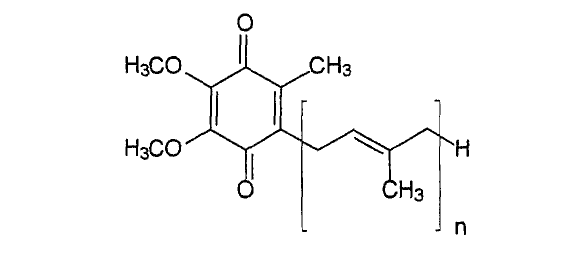 aus und stellen die am weitesten verbreiteten u. damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen u. Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

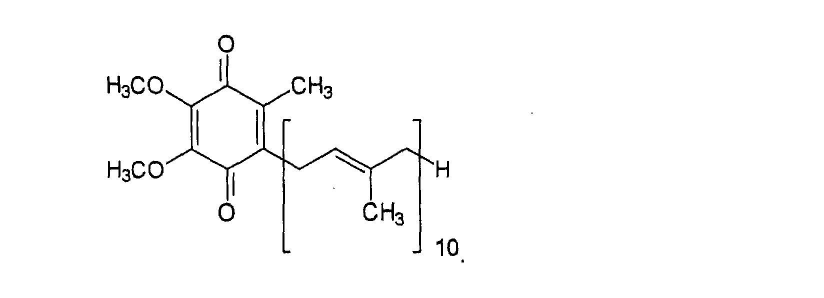

Plastochinone weisen die allgemeine Strukturformel 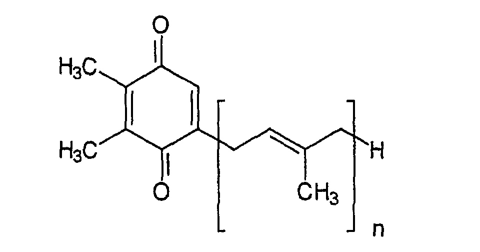 auf. Plastochinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Zusatzstoffe im Sinne der vorliegenden Erfindung. Kreatin zeichnet sich durch folgende Struktur aus:

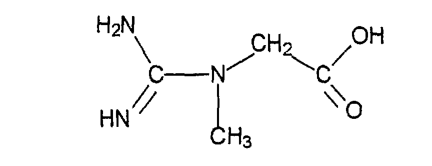

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Zusatzstoff ist L-Camitin [3-Hydroxy-4-(trimethylammonio)-buttersäurebetain]. Auch Acyl-Carnitine, welche gewählt aus der Gruppe der Substanzen der folgenden allgemeinen Strukturformel 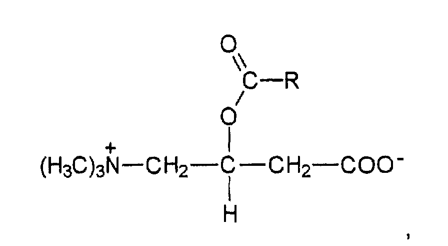 wobei R gewählt wird aus der Gruppe der verzweigten und unverzweigten Alkylreste mit bis zu 10 Kohlenstoffatomen sind vorteilhafte Zusatzstoffe im Sinne der vorliegenden Erfindung. Bevorzugt, sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Zusatzstoffe sind Sericosid, Pyridoxol, Vitamin K, Biotin und Aromastoffe.

Die Liste der genannten Zusatzstoffe bzw. Zusatzstoffkombinationen, die in den erfindungsgemäßen Zubereitungen verwendet werden können, soll selbstverständlich nicht limitierend sein. Die Zusatzstoffe können einzelnen oder in beliebigen Kombinationen miteinander verwendet werden.

Sodann können der Matrix des wirkstoffhaltigen Matrixpflasters pharmazeutisch wirksame Substanzen zugesetzt sein, besonders zu 0,1 bis 25 Gew.-%, ganz besonders zu 0,5 bis 10 Gew.-%.

Typische Wirkstoffe sind - ohne den Anspruch der Vollständigkeit im Rahmen der vorliegenden Erfindung zu erheben:

| **Indikation:** | **Wirkstoff** |
|---|---|
| Antimykotika | Nafitin |
| | Amorrolfin |
| | Tolnaftat |
| | Ciclopirox |
| Antiseptika | Thymol |
| | Eugenol |
| | Triclosan |
| | Hexachlorophen |
| | Benzalkoniumchlorid |
| | Clioquinol |
| | Chinolinol |
| | Undecensäure |
| | Ethacridin |
| | Chlorhexidin |
| | Hexetidin |
| | Dodicin |
| | lod |
| Nichtsteroidale Antirheumatika | Glykolsalicylat |
| | Flufenaminsäure |
| | Ibuprofen |
| | Etofenamat |
| | Ketoprofen |
| | Piroxicam |
| | Indomethacin |
| Antipuriginosa | Polidocanol |
| | Isoprenalin |
| | Crotamiton |
| Lokalanästhetika | Benzocain |
| Antipsoriatika | Ammoniumbitumasulfonat |
| Keratolytika | Harnstoff |
| | Salicylsäure |

Daneben können auch hyperämisierende Wirkstoffe wie natürliche Wirkstoffe des Cayenne-Pfeffers oder synthetische Wirkstoffe wie Nonivamid, Nicotinsäurederivate, bevorzugt Bencylnicotinat oder Propylnicotinat, genannt werden beziehungsweise Antiphlogistika und/oder Analgetika.

Bevorzugt erfolgt die Herstellung der wirkstoffhaltigen Matrix in einem Verfahren, bei dem alle Komponenten der haftklebrigen Matrix unter Verzicht auf den Zusatz von Lösungsmittel in der Schmelze homogenisiert werden.
Besonders bevorzugt werden alle Komponenten in einem kontinuierlichen oder diskontinuierlichen Prozeß bei einer Temperatur unterhalb von 100 °C verarbeitet.

Die Matrix zeichnet sich aus durch hervorragende Hafteigenschaften auf der Haut, durch eine leichte und schmerzfreie Wiederablösbarkeit sowie vor allem durch sein äußerst geringes Potential, Hautreizungen hervorzurufen. Der Herstellungsprozess verläuft unter vollständigem Verzicht von Lösungsmitteln.
Gegebenfalls kann die offene, auf die Haut zu applizierende Klebseite mit einer wiederablösbaren, abdeckenden Schutzschicht eingedeckt sein.

Bei der Auswahl der Füllstoffe wurde überraschenderweise gefunden, daß sich insbesondere Füllstoffe auf der Basis Siliziumdioxid oder von Cellulose eignen, wobei letztere eine isotrope Gestalt besitzen und bei Kontakt mit Wasser nicht zum quellen neigen. Dabei sind besonders Füllstoffe mit einer Partikelgröße von kleiner gleich 100 µm geeignet.
Der Einsatz hydrophiler Füllstoffe in einer unpolaren Matrix ist in der Literatur bekannt. Explizit für den Einsatz in transdermal therapeutischen Systemen werden Sie in EP 0 186 019 A1 beschrieben. Hier allerdings lediglich bis zu einer Konzentration von 3 bis 30 Gew. %, ohne daß Details zu diesen Füllstoffen erwähnt werden. Die Erfahrung zeigt, daß Systeme mit einem Füllstoffgehalt von über 30 Gew.-% deutlich an Klebrigkeit verlieren und hart und spröde werden. Dadurch verlieren sie die grundlegende Anforderung an ein transdermal therapeutisches System.
Im Rahmen der vorliegenden Erfindung konnte jedoch gezeigt werden, daß Füllstoffe auf der Basis von mikrokristalliner oder amorpher Cellulose dann in wesentlich höheren Konzentrationen ohne negative Beeinflussung der klebtechnischen Eigenschaften eingesetzt werden können, wenn sie eine isotrope Gestalt mit einer Partikelgröße von nicht größer als 100 µm besitzen. Höhere Gehalte an Füllstoffen sind zur Verbesserung der Trageeigenschaften insbesondere bei lange andauernder und wiederholter Anwendung wünschenswert.

Die aus den Materialien zur Stoma-Versorgung bekannten hydrophilen Füllstoffe sind in die erfindungsgemäße Matrix integriert, was zur Unterstützung der Hautverträglichkeit dient.

Das Ziel - die topische Applikation von Arzneistoffen aus der Gruppe der ätherischen Öle, die gegebenenfalls unter Einsatz sehr hautverträglicher Additive unterstützt werden - kann im Rahmen der vorliegenden Erfindung durch den Zusatz permeationsfördemder Hilfsstoffe wie zum Beispiel Fettsäureester unterstützt werden.

Überraschenderweise konnten die genannten Anforderungen insbesondere durch ein System realisiert werden, das neben Polyisobutylenen amorphe Poly-α-olefine in Kombination mit amorpher oder mikrokristalliner Cellulose enthält. Dieses einfache System enthält als Polymerbasis ausschließlich synthetische Inhaltsstoffe, deren Qualität sehr gut kontrollierbar ist. Dadurch können allergene Reaktionen weitgehend ausgeschlossen werden. Der vollständige Verzicht auf schlecht definierte Inhaltsstoffe wie zum Beispiel Naturkautschuk oder Klebharze führt dadurch zu besonders hautfreundlichen Matrizes. Die klebtechnischen Eigenschaften der erfindungsgemäßen Formulierung sind darüber hinaus sehr gut einstellbar. Des weiteren kann auf eine zusätzliche Additivierung des Systems zur Stabilisierung verzichtet werden.

Wie bereits angedeutet, können besonders hautfreundliche Systeme zur topischen Applikation ätherischer Öle auf der Basis von Polyisobutylenen unter Einsatz amorpher Poly-α-olefine sowie Cellulose-Partikel als Füllstoff realisiert werden.

Der besondere Vorteil dieser Rohstoffbasis besteht in der Verwendung ausschließlich voll gesättigter synthetischer Elastomere. Diese sind sehr gut definiert und charakterisiert, wodurch die Verunreinigung mit allergenen Begleitstoffen ausgeschlossen werden kann. Durch den hohen Sättigungsgrad sind diese eingesetzten Polymere sehr oxidationsstabil. Deshalb kann auf eine zusätzliche Additivierung mit Antoxidantien und sonstigen Stabilisatoren verzichtet werden. Eine solche Additivierung, wie sie beim Einsatz von Naturkautschuk beziehungsweise ungesättigter Synthesekautschuke notwendig ist, birgt aufgrund der chemischen Struktur der gebräuchlichen Additive immer die Gefahr einer Hautunverträglichkeit. Zusätzlich stellt sie einen zusätzlichen Kostenaufwand dar.
Des weiteren besitzen alle eingesetzten Elastomere je nach Höhe des Molekulargewichtes eine inhärente Klebrigkeit. Dadurch kann zusätzlich auf den Einsatz von Klebharzen verzichtet werden. Klebharze sind häufig auf der Basis von Kolophonium hergestellte Stoffgemische, die sehr schlecht definiert sind. Eine einheitliche Strukturformel kann in den seltensten Fällen angegeben werden. Dies erschwert den Einsatz von Klebharzen als Rohstoff in zulassungspflichtigen Arzneimitteln, wie im vorliegen Fall der transdermal therapeutischen Systeme.
Aufgrund des molekulargewichtsabhängigen Adhäsionsvermögen auf Haut sowohl der Polyisobutylene als auch der amorphen Poly-α-olefine lassen sich die klebtechnischen Eigenschaften des erfindungsgemäßen Systems in einem sehr weiten Bereich einstellen, ohne die Chemie der Basiskomponenten verändern zu müssen. Häufig genügt eine geringe Variation der prozentualen Verhältnisse der Grundkomponenten, um gewünschte Produkteigenschaften zu erhalten.
Dieser Aspekt ist vor allem innerhalb der Arzneimittelentwicklung sehr wichtig. Die sorgfältige Auswahl unbedenklicher und gut hautverträglicher Rohstoffe führt zu einem hohen Kosten- und Zeitaufwand. Es ist daher wünschenswert, Produkteigenschaften gezielt über Variation der prozentualen Zusammensetzung der bekannten Rohstoffe einstellen zu können. Der zeitaufwendige Austausch eines kompletten Rohstoffes wird dadurch vermieden.

### Beispiele

### Beispiele 1 bis 17

Zur Überprüfung des Einflusses verschiedener Inhaltsstoffe der haftklebrigen Matrix hinsichtlich des Haftungsvermögens auf Haut wurden 17 Vergleichsrezepturen im Rahmen eines statistischen Versuchsplans hergestellt.
Das Klebeverhalten der Massesysteme auf Haut wurde in einem Tragetest durch 6 freiwillige Probanden geprüft. Aus diesem Grund wurde zunächst auf die Einarbeitung des Arzneistoffes verzichtet. Bewertet wurden die Muster innerhalb eines Schulnotensystems auf einer Skala von 1 bis 6, wobei 1 die beste Bewertung darstellt, 6 die schlechteste Bewertung.

Die Labormuster wurden nach der folgenden allgemeinen Vorgehensweise hergestellt:

In einem mit Duplex-Schaufeln ausgestatteten Laborkneter wurde bei einer Temperatur von 100 °C die angegebene Menge Vistanex MM L80 vorgelegt und eine Stunde lang geknetet bis das Material krümelig ist. Anschließend wurden nacheinander die angegebenen Mengen Vistanex LM MH, Klebharz und amorphes Poly-α-olefin zugegeben und eine weitere Stunde geknetet, bis das Material homogen war. Abschließend wurde der Füllstoff in der angegebenen Menge zugegeben und weiterhin eine Stunde geknetet. Das Material wurde nach dem Abkühlen aus dem Kneter entfernt.
Anschließend wurde die Masse zwischen silikonisiertem Papier mit Hilfe einer Heißpresse bei ca. 120 °C auf eine Dicke von 500 µm ausgepreßt. Diese Muster wurden einseitig mit einer Rückenschicht aus Polypropylen laminiert und auf der dieser Schicht abgewandten Seite mit einer silikonisierten Polyester Folie abgedeckt.

Aus diesem Aufbau wurden ca. 2,0 x 6,0 cm² große Muster ausgestanzt, die die Form handelsüblicher Pflasterstrips besitzen.

Die so hergestellten Muster wurden durch die Probanden an der Innenseite der Unterarme aufgeklebt und über einen Zeitraum von 6 h getragen. Bewertet wurden der initiale tack der Muster auf Haut sowie das Haftungsvermögen der Muster über einen Zeitraum von 6 h.

Tabelle 1 enthält die nach dieser Vorgehensweise verarbeiteten Beispielrezepturen 1 bis 17.

**Tabelle 1:**

| Beispielrezepturen 1 bis 17 | | | | |
|---|---|---|---|---|
| | PIB I | PIB II | Modifizierendes Agens | Füllstoff |
| Beispiel 1 | 17,0 | 30,0 | 5,0 / I | 48,0 / I |
| Beispiel 2 | 20,0 | 50,0 | 5,0 / I | 25,0 / I |
| Beispiel 3 | 10,0 | 30,0 | 20,0 / II | 40,0 / I |
| Beispiel 4 | 10,0 | 50,0 | 20,0 / III | 20,0 / I |
| Beispiel 5 | 17,0 | 43,0 | 20,0 / III | 20,0 / I |
| Beispiel 6 | 7,0 | 38,0 | 5,0 / IV | 50,0 / I |
| Beispiel 7 | 10,0 | 30,0 | 20,0 / I | 40,0 / II |
| Beispiel 8 | 7,0 | 50,0 | 5,0 / II | 38,0 / II |
| Beispiel 9 | 20,0 | 50,0 | 5,0 / II | 25,0 / II |
| Beispiel 10 | 7,0 | 38,0 | 5,0 / III | 50,0 / II |
| Beispiel 11 | 17,0 | 43,0 | 20,0 / IV | 20,0 / II |
| Beispiel 12 | 20,0 | 30,0 | 20,0 / IV | 30,0 / II |
| Beispiel 13 | 7,0 | 50,0 | 20,0 / I | 23,0 / III |
| Beispiel 14 | 17,0 | 30,0 | 20,0 / II | 33,0 / III |
| Beispiel 15 | 20,0 | 30,0 | 5,0 / III | 45,0 / III |
| Beispiel 16 | 10,0 | 50,0 | 5,0 / IV | 35,0 / III |
| Beispiel 17 | 14,0 | 39,0 | 12,0 / IV | 35,0 / III |

Folgende Rohstoffe wurden verwendet:
- PIB I:: Polyisobutylen, Vistanex MM L80, Exxon Chemical
- PIB II:: Polyisobutylen, Vistanex LM MH, Exxon Chemical

Modifizierende Inhaltsstoffe:
- I:: Aliphatisch / Aromatisches Kohlenwasserstoffharz, Escorez 2101, Exxon Chemical
- II:: Hydriertes Polycyclopentadienharz, Escorez 5300 I, Exxon Chemical
- III:: Hotmelt Adhesive, Duro Tack H 1540, National Starch
- IV:: amorphes Poly-α-olefin, Eastoflex E 1003, Eastmann

### Füllstoffe:

- I:: Cellulose Fasern, Just Fiber, International Filler of Belgium
- II:: Mikrokristalline Cellulose, Avicel PH 101, FMC
- III:: Kolloidales Silica, HiSil, PPG Industries

Die klebtechnischen Eigenschaften auf Haut der so hergestellten Muster wurden nach einem Schulnotensystem von 1 bis 6 bewertet. Dabei stellt 1 die beste zu vergebende Note dar, 6 die schlechteste zu vergebende Note. Die Ergebnisse dieses Tragetests sind in Tabelle 2 zusammengestellt sowie in Abbildung 1 graphisch dargestellt.

**Tabelle 2:**

| Bewertung des Klebens auf Haut (./.: keine meßbare Adhäsion zu Haut) | | | | | | | | | | | | | | | | | |
|---|---|---|---|---|---|---|---|---|---|---|---|---|---|---|---|---|---|
| Rezeptur | 1 | 2 | 3 | 4 | 5 | 6 | 7 | 8 | 9 | 10 | 11 | 12 | 13 | 14 | 15 | 16 | 17 |
| Bewertung | 6 | 1,5 | 4,5 | 1 | 1 | 5,5 | 1 | 1 | 1 | 2 | 1 | 1 | 2 | 5,5 | ./. | 6 | 6 |

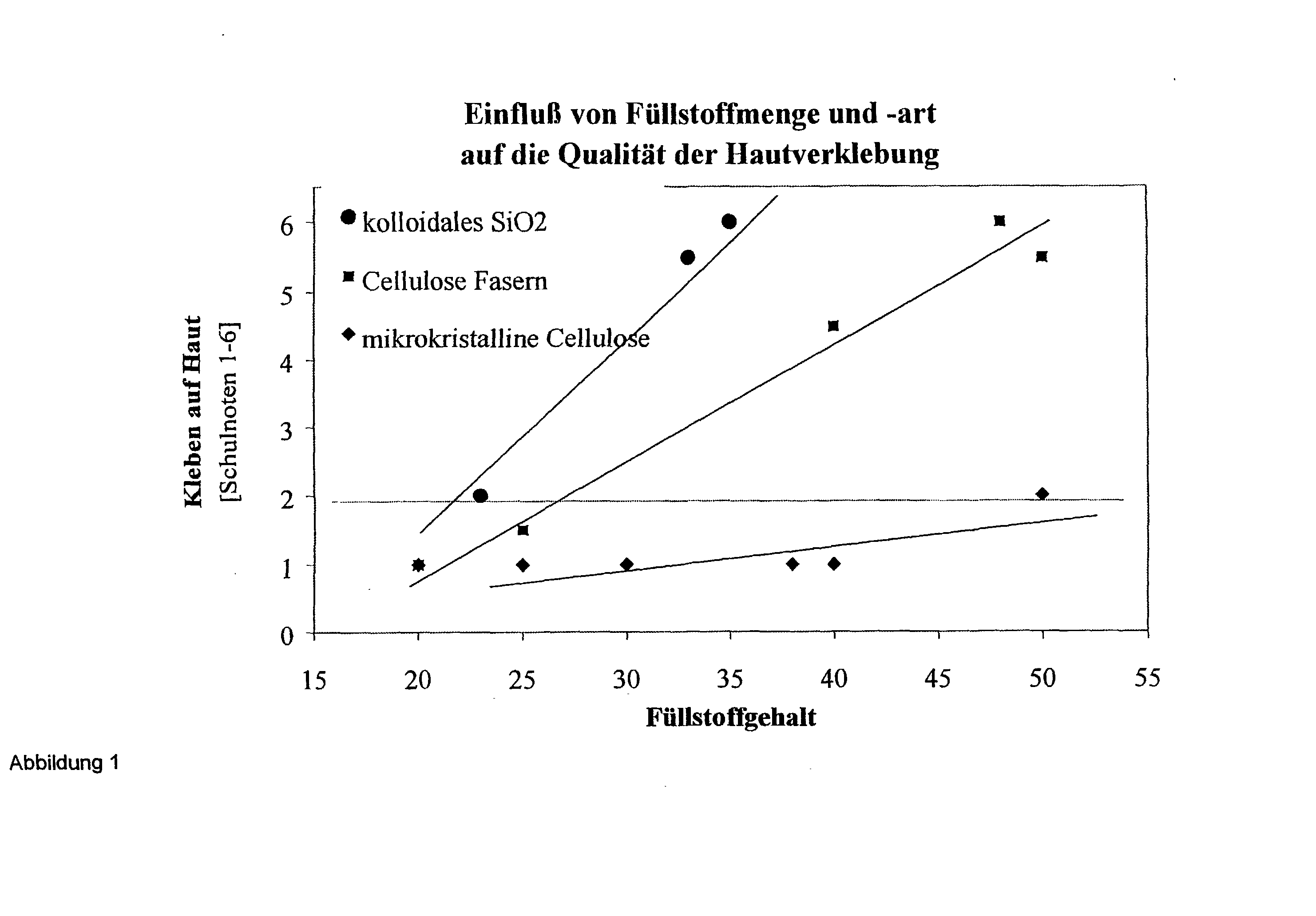

Aus der Abbildung ist deutlich zu erkennen, daß die Art und die Menge des Füllstoffes das Klebeverhalten der Systeme dominieren. Unterhalb einer Einsatzmenge von ca. 20 - 25 Gew.-% zeigen alle Systeme unabhängig vom eingesetzten Füllstoff ein mit mindestens "Gut" (2,0) bewertetes Klebeverhalten. Dies verändert sich drastisch, wenn die Füllstoffmenge auf über 30 Gew.-% angehoben wird.
Oberhalb einer Füllstoffmenge von 30 Gew.-% zeigen solche Systeme ein sehr gutes Klebeverhalten, in denen erfindungsgemäß Cellulose mit einer durchschnittlichen Partikelgröße von 50 µm eingesetzt werden.

### Beispiele 18 bis 29

Zur Überprüfung der Wirkstofffreisetzung eines erfindungsgemäßen Systems werden Labormuster basierend auf folgenden Rezepturen nach der allgemeinen Herstellbeschreibung präpariert.

### Beispiel 18

| | |
|---|---|
| Vistanex LM MH | 32,40 Gew.-% |
| Vistanex MM L80 | 16,80 Gew.-% |
| Eastoflex PLS E1003D | 6,70 Gew.-% |
| Avicel PH 101 | 39,10 Gew.-% |
| Menthol | 5,00 Gew.-% |

### Beispiel 19

| | |
|---|---|
| Vistanex LM MH | 43,33 Gew.-% |
| Vistanex MM L80 | 38,00 Gew.-% |
| Eastoflex PLS E1003D | 5,00 Gew.-% |
| Cetiol V | 12,67 Gew.-% |
| Pfefferminzöl | 1,00 Gew.-% |

### Beispiel 20

| | |
|---|---|
| Vistanex LM MH | 43,33 Gew.-% |
| Vistanex MM L80 | 38,00 Gew.-% |
| Eastoflex PLS E1003D | 5,00 Gew.-% |
| Cetiol V | 8,67 Gew.-% |
| Pfefferminzöl | 5,00 Gew.-% |

### Beispiel 21

| | |
|---|---|
| Vistanex LM MH | 48,33 Gew.-% |
| Vistanex MM L80 | 28,00 Gew.-% |
| Eastoflex PLS E1003D | 5,00 Gew.-% |
| Cetiol V | 8,67 Gew.-% |
| Pfefferminzöl | 10,00 Gew.-% |

### Beispiel 22

| | |
|---|---|
| Vistanex LM MH | 43,33 Gew.-% |
| Vistanex MM L80 | 38,00 Gew.-% |
| Eastoflex PLS E1003D | 5,00 Gew.-% |
| Cetiol V | 12,37 Gew.-% |
| Eukalyptusöl | 1,30 Gew.-% |

### Beispiel 23

| | |
|---|---|
| Vistanex LM MH | 43,33 Gew.-% |
| Vistanex MM L80 | 38,00 Gew.-% |
| Eastoflex PLS E1003D | 5,00 Gew.-% |
| Cetiol V | 8,67 Gew.-% |
| Eukalyptusöl | 5,00 Gew.-% |

### Beispiel 24

| | |
|---|---|
| Vistanex LM MH | 42,83 Gew.-% |
| Vistanex MM L80 | 38,00 Gew.-% |
| Eastoflex PLS E1003D | 5,00 Gew.-% |
| Cetiol V | 12,67 Gew.-% |
| Menthol | 1,00 Gew.-% |
| Bisabolol | 0,50 Gew.-% |

### Beispiel 25

| | |
|---|---|
| Vistanex LM MH | 42,83 Gew.-% |
| Vistanex MM L80 | 38,00 Gew.-% |
| Eastoflex PLS E1003D | 5,00 Gew.-% |
| Cetiol V | 12,67 Gew.-% |
| Menthol | 1,00 Gew.-% |
| Vitamin E | 0,50 Gew.-% |

### Beispiel 26

| | |
|---|---|
| Vistanex LM MH | 42,83 Gew.-% |
| Vistanex MM L80 | 38,00 Gew.-% |
| Eastoflex PLS E1003D | 5,00 Gew.-% |
| Cetiol V | 12,67 Gew.-% |
| Menthol | 1,00 Gew.-% |
| Vitamin F | 0,50 Gew.-% |

### Beispiel 27

| | |
|---|---|
| Vistanex LM MH | 40,33 Gew.-% |
| Vistanex MM L80 | 36,00 Gew.-% |
| Eastoflex PLS E1003D | 5,00 Gew.-% |
| Cetiol V | 12,67 Gew.-% |
| Menthol | 1,00 Gew.-% |
| Kamille | 5,00 Gew.-% |

### Beispiel 28

| | |
|---|---|
| Vistanex LM MH | 40,33 Gew.-% |
| Vistanex MM L80 | 36,00 Gew.-% |
| Eastoflex PLS E1003D | 5,00 Gew.-% |
| Cetiol V | 12,67 Gew.-% |
| Menthol | 1,00 Gew.-% |
| Jojoba | 5,00 Gew.-% |

### Beispiel 29

| | |
|---|---|
| Vistanex LM MH | 35,00 Gew.-% |
| Vistanex MM L80 | 35,00 Gew.-% |
| Eastoflex PLS E1003D | 5,00 Gew.-% |
| Cetiol V | 5,00 Gew.-% |
| Pfefferminzöl | 5,00 Gew.-% |
| Methylsalicylat | 10,00 Gew.-% |
| Kampfer | 5,00 Gew.-% |

## Patentansprüche

1. Wirkstoffhaltiges Matrixpflaster zur kontrollierten Abgabe von ätherischen Ölen an die Haut und/oder die Atemluft, bestehend aus einer flexiblen Deckschicht und einer wirkstoffhaltigen, wasserunlöslichen haftklebrigen Matrix, wobei die haftklebrige Matrix frei von Mineralölen und Klebharzen ist und aufgebaut ist aus
a) synthetischen Gerüstpolymeren auf der Basis von Polyisobutylen zu 25 bis 90 Gew.-%,
b) amorphem Poly-α-olefin zu 5 bis 30 Gew.-%,
c) aus einem unlöslichen, bevorzugt hydrophilen Füllstoff zu 0 bis 60 Gew.-% und
d) ätherischen Ölen zu 0,5 bis 25 Gew.-%.

2. Wirkstoffhaltiges Matrixpflaster nach Anspruch 1, **dadurch gekennzeichnet, daß** die Matrix Poly-α-olefine in einer Konzentration von 5 bis 10 Gew.-% enthält.

3. Wirkstoffhaltiges Matrixpflaster nach Ansprüchen 1 und 2, **dadurch gekennzeichnet, daß** sich das Polyisobutylen zusammensetzt aus
hochmolekularem PIB zu 5 bis 55 Gew.-% und
niedermolekularem PIB zu 20 bis 60 Gew.-%.

4. Wirkstoffhaltiges Matrixpflaster nach Ansprüchen 1 bis 3, **dadurch gekennzeichnet, daß** der Füllstoff auf Cellulose sowie seinen Derivaten basiert.

5. Wirkstoffhaltiges Matrixpflaster nach Ansprüchen 1 bis 4, **dadurch gekennzeichnet, daß** die Matrix bevorzugt einen hydrophilen Füllstoff auf der Basis von Cellulose sowie seinen Derivaten enthält, deren mittlere Korngröße im Bereich von 20 bis 60 µm beträgt.

6. Wirkstoffhaltiges Matrixpflaster nach Ansprüchen 1 bis 5, **dadurch gekennzeichnet, daß** die Matrix bevorzugt 1 bis 10 Gew.-% ätherische Öle enthält, insbesondere gewählt aus der Gruppe Eucalyptusöl, Pfefferminzöl, Kamillenöl, Campher, Menthol, Citrusöl, Zimtöl, Thymianöl, Lavendelöl, Nelkenöl, Teebaumöl, Cajeputöl, Niaouliöl, Kanukaöl, Manukaöl, Latschenkieferöl.

7. Wirkstoffhaltiges Matrixpflaster nach Ansprüchen 1 bis 6, **dadurch gekennzeichnet, daß** der Matrix permeationsfördernde Inhaltsstoffe im Konzentrationsbereich bis zu 30 Gew.-%, bevorzugt 5 bis 15 Gew.-% zugesetzt werden.

8. Wirkstoffhaltiges Matrixpflaster nach Ansprüchen 1 bis 7, **dadurch gekennzeichnet, daß** der Matrix hautpflegende, kosmetische Zusatzstoffe zugesetzt sind, besonders zu 0,2 bis 10 Gew.-%, ganz besonders zu 0,5 bis 5 Gew.-%.

9. Wirkstoffhaltiges Matrixpflaster nach Ansprüchen 1 bis 8, **dadurch gekennzeichnet, daß** der Matrix pharmazeutisch wirksame Substanzen zugesetzt sind, besonders zu 0,1 bis 25 Gew.-%, ganz besonders zu 0,5 bis 10 Gew.-%.

10. Verfahren zur Herstellung eines wirkstoffhaltigen Matrixpflasters nach Ansprüchen 1 bis 9, **dadurch gekennzeichnet, daß** alle Komponenten der haftklebrigen Matrix unter Verzicht auf den Zusatz von Lösungsmittel in der Schmelze homogenisiert werden.

11. Verfahren zur Herstellung eines wirkstoffhaltigen Matrixpflasters nach Anspruch 10, **dadurch gekennzeichnet, daß** alle Komponenten in einem kontinuierlichen oder diskontinuierlichen Prozeß bei einer Temperatur unterhalb von 100 °C verarbeitet werden.

## Claims

1. Active substance matrix patch for the controlled delivery of essential oils to the skin and/or the breath, comprising a flexible outer layer and a water-insoluble pressure-sensitively adhesive matrix which contains active substance, the pressure sensitive matrix being free from mineral oils and tackifier resins and being composed of
a) synthetic framework polymers based on polyisobutylenes, at from 25 to 90% by weight,
b) amorphous poly-α-olefin at from 5 to 30% by weight,
c) an insoluble, especially hydrophilic, filler at from 0 to 60% by weight, and
d) essential oils at from 0.5 to 25% by weight.

2. Active substance matrix patch according to Claim 1, **characterized in that** the matrix contains poly-α-olefins in a concentration of from 5 to 10% by weight.

3. Active substance matrix patch according to Claims 1 and 2, **characterized in that** the polyisobutylene is composed of
high molecular weight PIB at from 5 to 55% by weight and
low molecular weight PIB at from 20 to 60% by weight.

4. Active substance matrix patch according to Claims 1 to 3, **characterized in that** the filler is based on cellulose and its derivatives.

5. Active substance matrix patch according to Claims 1 to 4, **characterized in that** the matrix preferably comprises a hydrophilic filler based on cellulose and its derivatives whose average particle size is in the range from 20 to 60 µm.

6. Active substance matrix patch according to Claims 1 to 5, **characterized in that** the matrix contains preferably from 1 to 10% by weight of essential oils selected in particular from the group consisting of eucalyptus oil, peppermint oil, camomile oil, camphor, menthol, citrus oil, cinnamon oil, thyme oil, lavender oil, clove oil, tea tree oil, cajeput oil, niaouli oil, kanuka oil, manuka oil, and dwarf pine oil.

7. Active substance matrix patch according to Claims 1 to 6, **characterized in that** permeation promoters in the concentration range up to 30% by weight, preferably from 5 to 15% by weight, are added to the matrix.

8. Active substance matrix patch according to Claims 1 to 7, **characterized in that** cosmetic skincare additives are added to the matrix, in particular at from 0.2 to 10% by weight, very particularly at from 0.5 to 5% by weight.

9. Active substance matrix patch according to Claims 1 to 8, **characterized in that** pharmaceutically active substances are added to the matrix, in particular at from 0.1 to 25% by weight, very particularly at from 0.5 to 10% by weight.

10. Process for producing an active substance matrix patch according to Claims 1 to 9, **characterized in that** all components of the pressure sensitive matrix are homogenized in the melt with the omission of solvent.

11. Process for producing an active substance matrix patch according to Claim 10, **characterized in that** all components are processed in a continuous or batchwise operation at a temperature below 100°C.

## Revendications

1. Pansement à matrice contenant une substance active pour la libération contrôlée d'huiles essentielles dans la peau et/ou l'air inhalé, constitué par une couche de recouvrement souple et d'une matrice auto-adhésive contenant une substance active, insoluble dans l'eau, la matrice auto-adhésive étant exempte d'huiles minérales et de résines adhésives et étant constituée par
a) des polymères de structure synthétiques à base de polyisobutylène à raison de 25 à 90% en poids
b) une poly-α-oléfine amorphe à raison de 5 à 30% en poids
c) une charge insoluble, de préférence hydrophile à raison de 0 à 60% en poids et
d) des huiles essentielles à raison de 0,5 à 25% en poids.

2. Pansement à matrice contenant une substance active selon la revendication 1, **caractérisé en ce que** la matrice contient des poly-α-oléfines en une concentration de 5 à 10% en poids.

3. Pansement à matrice contenant une substance active selon les revendications 1 et 2, **caractérisé en ce que** le polyisobutylène est constitué par du PIB de haut poids moléculaire à raison de 5 à 55% en poids et du PIB de bas poids moléculaire à raison de 20 à 60% en poids.

4. Pansement à matrice contenant une substance active selon les revendications 1 à 3, **caractérisé en ce que** la charge est à base de cellulose et ses dérivés.

5. Pansement à matrice contenant une substance active selon les revendications 1 à 4, **caractérisé en ce que** la matrice contient de préférence une charge hydrophile à base de cellulose ainsi que ses dérivés, dont la granulométrie moyenne se situe dans la plage de 20 à 60 µm.

6. Pansement à matrice contenant une substance active selon les revendications 1 à 5, **caractérisé en ce que** la matrice contient de préférence 1 à 10% en poids d'huiles essentielles, en particulier choisies dans le groupe de l'essence d'eucalyptus, l'essence de menthe, l'essence de camomille, le camphre, le menthol, l'essence d'agrumes, l'essence de cannelle, l'essence de thym, l'essence de lavande, l'essence de girofle, l'essence de l'arbre à thé, l'essence de cajeput, l'essence de niaouli, l'essence de kanuka, l'essence de manuka, l'essence de pin.

7. Pansement à matrice contenant une substance active selon les revendications 1 à 6, **caractérisé en ce qu'**on ajoute à la matrice des constituants favorisant la perméation dans une plage de concentration allant jusqu'à 30% en poids, de préférence de 5 à 15% en poids.

8. Pansement à matrice contenant une substance active selon les revendications 1 à 7, **caractérisé en ce qu'**on a ajouté à la matrice des additifs cosmétiques de soin de la peau, en particulier à raison de 0,2 à 10% en poids, tout particulièrement à raison de 0,5 à 5% en poids.

9. Pansement à matrice contenant une substance active selon les revendications 1 à 8, **caractérisé en ce qu'**on a ajouté à la matrice des substances pharmaceutiquement actives, en particulier à raison de 0,1 à 25% en poids, tout particulièrement à raison de 0,5 à 10% en poids.

10. Procédé pour la préparation d'un pansement à matrice contenant une substance active selon les revendications 1 à 9, **caractérisé en ce que** tous les composants de la matrice auto-adhésive sont homogénéisés dans la masse fondue en renonçant à l'addition de solvants.

11. Procédé pour la préparation d'un pansement à matrice contenant une substance active selon la revendication 10, **caractérisé en ce que** tous les composants sont transformés dans un procédé continu ou discontinu à une température inférieure à 100°C.
